# EUROPEAN PATENT APPLICATION

(11) **EP 0 930 072 A1**
(43) Date of publication of application: **21.07.1999**
(21) Application number: 98300193.4
(22) Date of filing: 13.01.1998
(51) Int. Cl.: A61K 33/00, A61K 33/04, A61K 33/24, A61K 33/26, A61K 33/30, A61K 33/32

(54) **Vitamin and mineral containing compositions for the treatment of dry eye**

(71) Applicant: Lalvani, Kartar, Dr., Wembley, Middlesex HA0 1NU (GB); Seoane Sanchez, José Francisco, 15011 La Coruna (ES); Taylor, Robert P., London W11 1EP (GB)
(72) Inventor: Lalvani, Kartar, Dr., Wembley, Middlesex HA0 1NU (GB); Seoane Sanchez, José Francisco, 15011 La Coruna (ES); Taylor, Robert P., London W11 1EP (GB)

(57) **Abstract**

This invention relates to the use of novel combinations of vitamins and minerals to give a product which affects an improvement in conjunctival goblet cell population density, improving the mucus layer of the tear film to mediate the uniform spread of the aqueous phase, and acts to promote an absolute increase in pre-ocular tear film stability in dry eye conditions.

The invention involves the systemic use of unique mixtures of specific metallic cations, non-metallic elements and vitamins and provides greater convenience than existing treatments which require the frequent application of topical solutions or lubricant ointments.

The action by the invention to improve tear stability is of particular importance to the treatment of dry eye conditions experienced by contact lens users.

## Description

This invention relates to a novel combination of nutritional factors which provide a systemic treatment for marginal dry eye conditions, when occurring as a result of a decrease in tear secretion or quality, especially during contact lens use. Typical symptoms include dryness, scratchiness, stinging and mucus formation. While idiopathic dry eye (Keratoconjunctivitis Sicca) has been estimated to effect 10 million patients in the USA alone, over 30 million Americans may exhibit marginal symptoms of dry eye, which can make the use of contact lenses uncomfortable.

There are three layers of tear film which lubricate the eye. The outer oily layer smooths the tear surface and reduces the evaporation of the tears. The middle watery tear layer cleanses the eye and washes away foreign particles or irritants. The inner mucus layer, produced by the goblet cells of the conjunctiva, allows the tear to spread evenly over the surface of the eye. Ocular comfort depends on adequate amounts of the three secretions and their correct interaction. Rupture of the tear film exposes the cornea to the atmosphere which can damage the sensitive surface. An unstable or inadequate tear film can be due to insufficient tears, but is more commonly caused by poor wetting of the eye surface by the tears caused by disruption of the underlying mucus layer.

The condition is typically age related, and contact lens wearers and post-menopausal women comprise the groups most commonly affected by eye discomfort. Other factors that contribute to dry eye include central heating, wind, cigarette smoke and pollution. Dry eye is also associated with autoimmune diseases, such as rheumatoid arthritis and Sjogren's syndrome.

No satisfactory treatments for dry eye conditions are currently in use. The use of topic gels or lubricant ointments which are formulated with sodium chloride or other salts to mimic natural tears provide some limited relief but this is short-lived and topical application is inconvenient. 'Artificial tears' often do not wet the affected eye surface completely, and their oncotic pressure is too low to assist effectively in the healing of the damaged ocular surface.

The present invention relates to the use of appropriate combinations of vitamins and minerals to give a product with a balance of ingredients with one or both of the following actions;
1. To promote an absolute increase in pre-ocular tear film stability.
2. To effect an improvement in conjunctival goblet cell population density, improving the mucus layer to mediate the uniform spread of the aqueous phase.

The above action may produce an increase in conjunctival health, in terms of squamous metaplasia.

The invention does not involve the direct application of 'artificial tears', or ointments. Oral administration of the invention provides greater convenience than existing treatments which may require the direct application of solution several times a day, or as often as every hour. The problem of allergic reactions of the eye to preservatives contained in artificial solutions is also avoided by the systemic action of the invention.

Improvement in tear stability by the invention is of particular importance to contact lens users or those exposed to prolonged use of visual display units (VDU's). Both of these groups are particularly susceptible to marginal dry eye.

The products of the invention are composed of mixtures of one or more of the metals zinc, iron, copper, magnesium, manganese, selenium and chromium as cations and the non-metal elements selenium and iodine as anions, in various proportions. To these components vitamin D, vitamin E, vitamin A, vitamin K, vitamin B-complex and synthetic or natural betacarotene may be added to form the final product.

The proportion of various ingredients by elemental weight, relative to selenium considered as unity, are in the ranges:

For the metal cations of Cu⁺⁺ (0-150), Fe⁺⁺ (0-3,000), Zn⁺⁺ (0-1,200), Mn⁺⁺ (0-500), Cr (0.5-4) and Mg⁺⁺ (0-15,000) in the form of their oxides or their salts involving the anions chloride, bromide, sulphate, carbonate, phosphate, and also their hydroxy derivatives, plus the non-metal iodine in the form of I⁻ (0-25) as an anion in salts with sodium and potassium cations.

To the above the following may be optionally added, in proportions relative to that of selenium; Vitamin A as retinol equivalents (0-150), Vitamin C (0-60,000), vitamin D (0-0.5), vitamin E (0-5,000), vitamin K (0-5), pantothenic acid (0-4,000), and betacarotene (0-400). The B group vitamins in the proportions relative to selenium are in the range B1 (0-2,000), B2 (0-4,500), B3 (0-3,500), B6 (0-2,000), folic acid (0-10), B12 (0-1.5).

If selenium is omitted from the mixture then the mean of the range of one of the other metals may be used as the standard.

A typical product is constituted as follows:

### Example 1

| | |
|---|---|
| Vitamin A | 300 mcg |
| Betacarotene | 3 mg |
| Vitamin E | 60 mg |
| Vitamin C | 150 mg |
| Vitamin D | 2.5 mcg |
| Vitamin B6 (Pyridoxine) | 4.5 mg |
| Vitamin B1 (Thiamin Mononitrate) | 7.5 mg |
| Vitamin B2 (Riboflavin) | 5 mg |
| Vitamin B12 (Cyanocobalamin) | 4.5 mcg |
| Folic Acid | 250 mcg |
| Vitamin K1 | 100 mcg |
| Calcium Pantothenate | 10 mg |
| Zinc | 7.5 mg |
| Iron | 3 mg |
| Copper | 1 mg |
| Magnesium | 50 mg |
| Manganese | 2 mg |
| Selenium | 100 mcg |
| Chromium | 50 mcg |
| Iodine | 100 mcg |

### Example 2

| | |
|---|---|
| Vitamin E | 120 mg |
| Vitamin C | 300 mg |
| Vitamin D | 5 mcg |
| Vitamin B6 (Pyridoxine) | 30 mg |
| Vitamin B1 (Thiamin Mononitrate) | 15 mg |
| Vitamin B2 (Riboflavin) | 10 mg |
| Vitamin B12 (Cyanocobalamin) | 9 mcg |
| Folic Acid | 500 mcg |
| Vitamin K1 | 200 mcg |
| Calcium Pantothenate | 20 mg |
| Zinc | 15 mg |
| Iron | 6 mg |
| Copper | 2 mg |
| Magnesium | 100 mg |
| Manganese | 4 mg |
| Selenium | 200 mcg |
| Chromium | 100 mcg |
| Iodine | 200 mcg |
| Bioflavonoids | 30 mg |
| Mixed carotenoids | 6 mg |

The example is given by way of illustration only, and is not constructed as limiting the invention in scope or in spirit, as many modifications will be apparent from disclosure to those in this art. Products may be combined with herbal and other natural materials, preferably bioflavonoids (1-80mg), amino acids, or mixed carotenoids (1-40mg), including alpha carotene, cryptoxanthin, lutein and zeaxanthin.

The invention involves the systemic delivery of ingredients in any pharmaceutical form, for example, tablet, capsule, liquid, patch, or injection.

## Claims

1. A product for the treatment of dry eye and marginal dry eye conditions in animals and humans comprising selenium (elemental 1-500mcg), and betacarotene (1-50mg).

2. Products as claimed in claim 1, with the addition of one or more B group vitamins.

3. Products as claimed in claim 1 with the addition of 1-500mcg chromium as an anion.

4. Products as claimed in claim 2 with the addition of 1-500mcg chromium as an anion.

5. Product as claimed in claim 3 with the addition of 1-200mg iron as a cation.

6. Product as claimed in claim 4 with the addition of 1-200mg iron as a cation.

7. Products as claimed in claim 1 with the addition of 1-75mg zinc as a cation.

8. Products as claimed in claim 2 with the addition of 1-75mg zinc as a cation.

9. Products as claimed in claim 3 with the addition of 1-75mg zinc as a cation.

10. Products as claimed in claim 4 with the addition of 1-75mg zinc as a cation.

11. Products as claimed in claim 5 with the addition of 1-75mg zinc as a cation.

12. Products as claimed in claim 6 with the addition of 1-75mg zinc as a cation.

13. Products as claimed in claim 1 with the addition of 0.5-40mg manganese as a cation.

14. Products as claimed in claim 2 with the addition of 0.5-40mg manganese as a cation.

15. Products as claimed in claim 4 with the addition of 0.5-40mg manganese as a cation.

16. Products as claimed in claim 6 with the addition of 0.5-40mg manganese as a cation.

17. Products as claimed in claim 8 with the addition of 0.5-40mg manganese as a cation.

18. Products as claimed in claim 12 with the addition of 0.5-40mg manganese as a cation.

19. A product for the treatment of dry eye and marginal dry eye conditions in animals and humans comprising zinc (elemental 1-75mg), and vitamin C (1-4,000mg).

20. Products as claimed in claim 19 with the addition of 1-500mcg selenium as an anion.

21. Product as claimed in claim 19 with the addition of 1-200mg iron as a cation.

22. Product as claimed in claim 20 with the addition of 1-200mg iron as a cation.

23. Product as claimed in claim 22 with the addition of one or more B group vitamins.
